# EUROPÄISCHE PATENTSCHRIFT

(11) **EP 3 477 784 B1**
(45) Veröffentlichungstag und Bekanntmachung des Hinweises auf die Patenterteilung: **28.09.2022**
(21) Anmeldenummer: 18198890.8
(22) Anmeldetag: 05.10.2018
(51) Int. Cl.: H01R 13/187, H01R 13/24

(54) **HANDHABUNGSEINRICHTUNG FÜR EIN MIKROINVASIVES MEDIZINISCHES INSTRUMENT**
HANDLE FOR A MICROINVASIVE MEDICAL INSTRUMENT
DISPOSITIF DE TRAITEMENT POUR UN INSTRUMENT MÉDICAL MICROINVASIF

(30) Priorität: 24.10.2017 DE 102017124775
(43) Veröffentlichungstag der Anmeldung: 01.05.2019
(73) Patentinhaber: Karl Storz SE & Co. KG, 78532 Tuttlingen (DE)
(72) Erfinder: Wittke, Uwe, 78532 Tuttlingen (DE)

(56) Entgegenhaltungen:
- EP-A1- 1 557 913
- EP-A2- 2 653 120
- WO-A2-99/03405
- DE-A1-102014 116 065
- US-B1- 6 238 394

## Beschreibung

Die vorliegende Erfindung ist auf eine Handhabungseinrichtung für ein mikroinvasives medizinisches Instrument und ein mikroinvasives medizinisches Instrument mit einer solchen Handhabungseinrichtung bezogen.

Ein typisches mikroinvasives medizinisches Instrument umfasst eine Handhabungseinrichtung an seinem proximalen Ende, einen langen und in der Regel dünnen Schaft, der sich von dem proximalen Ende zu dem distalen Ende des Instruments erstreckt, und einem Werkzeug oder einer anderen Wirkeinrichtung zum Greifen, Quetschen, Koagulieren, Schneiden, Stanzen oder für andere Wirkungen an dem distalen Ende des Instruments. In dem Schaft verlaufen eine oder mehrere Übertragungseinrichtungen zum Übertragen einer Kraft und/oder eines Drehmoments von der Handhabungseinrichtung am proximalen Ende zu der Wirkeinrichtung am distalen Ende. Bei mikroinvasiven medizinischen Instrumenten mit elektrochirurgischer Funktion, insbesondere bei bipolaren elektrochirurgischen mikroinvasiven medizinischen Instrumenten ist die Übertragungseinrichtung oft ferner an der Übertragung elektrischer Leistung von dem proximalen Ende zu dem distalen Ende beteiligt.

Hochwertige mikroinvasive medizinische Instrumente sind in der Regel wiederverwendbar. Um eine Reinigung nach der Verwendung zu vereinfachen, einen Austausch einer defekten Komponente und eine alternative Verwendung unterschiedlicher Komponenten (beispielsweise unterschiedlicher Wirkeinrichtungen oder von Schäften unterschiedlicher Länge) zu ermöglichen, ist ein hochwertiges mikroinvasives medizinisches Instrument in der Regel zerlegbar. Bei einem zerlegbaren medizinischen Instrument, bei dem die Übertragungseinrichtung auch an der Übertragung elektrischer Leistung beteiligt ist, muss insbesondere am proximalen Ende des Instruments ein mechanisch trennbarer elektrischer Kontakt zu der Übertragungseinrichtung hergestellt werden.

In DE 43 23 584 A1 ist ein zerlegbares medizinisches Instrument mit einem Elektrodenrohr 11 beschrieben. Eine Kontaktfeder 22 liegt in axialer Richtung an dem hinteren Ende des Elektrodenrohrs elektrisch leitend an und ist mit einem Hochspannungs-Zuleitungskabel 23 elektrisch leitend verbunden (Spalte 3, Zeilen 11 bis 14).

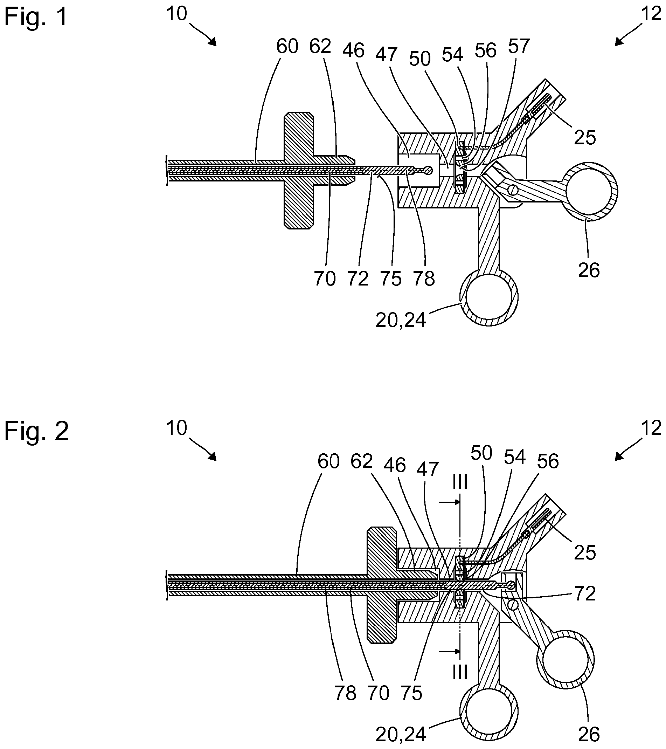

In DE 91 90 051.4 U1 ist ein Handstück 1, das elektrochirurgisches Schneiden durchführen kann, beschrieben (Seite 7, vorletzter Absatz). Eine elektrisch leitfähige Verbindung zu einem Werkzeug 6 (Figur 1) wird über eine elektrisch leitfähige Klemme 17, ein elektrisch leitfähiges Metallband 18 und einen elektrisch leitfähigen O-Ring 19 hergestellt (Seite 10, letzter Absatz; Figur 2).

In DE 195 12 640 C2 ist ein chirurgisches Endoskopieinstrument mit Arbeitselektrode beschrieben. Ein Handhabungsteil 2 des Endoskopieinstruments weist zwei von einander isolierte Kontaktfedern 18 zur elektrischen Kontaktierung je eines korrespondierenden Kontaktstücks 14 am proximalen Ende eines mit dem Handhabungsteil 2 zu verbindenden Schaftteils 1 auf (Spalte 3, Zeilen 20 bis 24; Figuren).

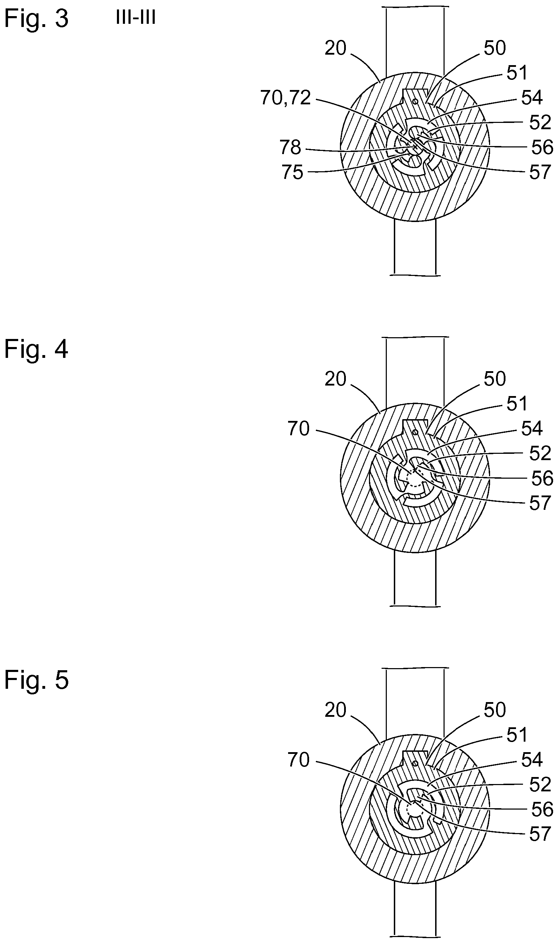

In DE 103 17 038 A1 ist ein elektrochirurgisches Instrument beschrieben. Ein Versorgungskabel 22 umfasst einen Draht 56, der an einen ersten Elektrodenkontakt 60 gebondet ist, und einen Draht 58, der an einen zweiten Elektrodenkontakt 62 gebondet ist (Spalte 5, Zeilen 25 bis 30; Figur 5).

In CN 201529146 U ist ein elektrochirurgisches Instrument beschrieben. Ein Kontaktstift 15 berührt ein proximales Ende eines Schafts (Figur 2).

In EP 2 303 170 B1 ist eine Katheteranordnung beschrieben. Eine Handhabungseinrichtung weist eine Mehrzahl von Kontakten 146 oder Einstellschrauben 148 auf, die je einen Ringkontakt 150 elektrisch kontaktieren (Absätze [0026], [0033]; Figuren 7, 8).

DE 10 2014 116065 offenbart den Oberbegriff des Anspruchs 1 .

Eine Aufgabe der vorliegenden Erfindung besteht darin, eine verbesserte Handhabungseinrichtung und ein verbessertes mikroinvasives medizinisches Instrument zu schaffen, die insbesondere einen zuverlässigen elektrischen Kontakt zu einer Übertragungseinrichtung des mikroinvasiven medizinischen Instruments ermöglicht.

Diese Aufgabe wird durch die Gegenstände der unabhängigen Ansprüche gelöst. Weiterbildungen sind in den abhängigen Ansprüchen angegeben.

Eine Handhabungseinrichtung für ein mikroinvasives medizinisches Instrument umfasst eine Ausnehmung zum Aufnehmen eines proximalen Bereichs einer elektrisch leitfähigen Übertragungseinrichtung zum Übertragen von elektrischer Leistung oder eines elektrischen Signals und zumindest entweder einer Kraft oder eines Drehmoments zu einem distalen Ende eines mikroinvasiven medizinischen Instruments und eine Kontaktierungseinrichtung zum Herstellen eines elektrischen Kontakts zu einer in der Ausnehmung angeordneten Übertragungseinrichtung, wobei die Kontaktierungseinrichtung mehrere Kontaktflächen zum gleichzeitigen Anliegen an einer Oberfläche einer in der vorgesehenen Weise in der Ausnehmung angeordneten Übertragungseinrichtung aufweist.

Die Handhabungseinrichtung ist vorgesehen und ausgebildet, um zusammen mit einem Schaft, insbesondere einem Außenschaft, einer Übertragungseinrichtung (und optional weiteren Übertragungseinrichtungen) und einem Werkzeug oder einer anderen Wirkeinrichtung ein mikroinvasives medizinisches Instrument zu bilden. Die Handhabungseinrichtung bildet also eine von mehreren Komponenten eines mikroinvasiven medizinischen Instruments. Der Schaft, die Übertragungseinrichtung, die Wirkeinrichtung und ggf. weitere Komponenten des mikroinvasiven medizinischen Instruments sind insbesondere nicht Bestandteil der Handhabungseinrichtung.

Die Handhabungseinrichtung umfasst insbesondere ein feststehendes Bauteil zur mechanischen Verbindung mit einem proximalen Ende eines Außenschafts oder eines anderen Schafts für ein mikroinvasives medizinisches Instrument. Das feststehende Bauteil ist insbesondere zerstörungsfrei lösbar oder sogar ohne Verwendung von Werkzeug zerstörungsfrei lösbar mit einem Schaft mechanisch verbunden oder verbindbar. Alternativ ist das feststehende Bauteil nicht zerstörungsfrei lösbar oder nur unter Verwendung von Werkzeug zerstörungsfrei lösbar mit einem Schaft verbindbar oder verbunden. Das feststehende Bauteil ist insbesondere insofern feststehend als es mit einem Schaft starr oder relativ zu diesem lediglich um dessen Längsachse rotierbar mechanisch verbindbar oder verbunden ist.

Die Handhabungseinrichtung umfasst insbesondere ferner ein relativ zu dem feststehenden Bauteil manuell bewegbares, beispielsweise um eine Schwenkachse schwenkbares Bauteil. Die Schwenkachse ist insbesondere orthogonal zur Längsachse eines in der vorgesehenen Weise mit der Handhabungseinrichtung verbundenen Schafts. Das manuell bewegbare Bauteil ist zur zerstörungsfrei lösbaren und insbesondere ohne Verwendung von Werkzeug lösbaren mechanischen Kopplung mit einer Übertragungseinrichtung für ein mikroinvasives medizinisches Instrument vorgesehen und ausgebildet. Alternativ ist das manuell bewegbare Bauteil auf eine nicht oder nur unter Verwendung von Werkzeug zerstörungsfrei lösbare Weise mit der Übertragungseinrichtung gekoppelt.

Die Ausnehmung zum Aufnehmen eines proximalen Bereichs einer Übertragungseinrichtung kann so angeordnet und ausgebildet sein, dass das proximale Ende der Übertragungseinrichtung aus der Handhabungseinrichtung, insbesondere aus deren feststehendem Bauteil proximal heraus ragt. Der von der Ausnehmung aufzunehmende Bereich einer Übertragungseinrichtung muss also das proximale Ende der Übertragungseinrichtung nicht umfassen, befindet sich aber nahe dem proximalen Ende der Übertragungseinrichtung. Der Querschnitt der Ausnehmung zum Aufnehmen des proximalen Bereichs der Übertragungseinrichtung ist insbesondere an den Querschnitt des proximalen Bereichs der Übertragungseinrichtung angepasst.

Die Handhabungseinrichtung umfasst insbesondere ferner eine Ausnehmung zum Aufnehmen eines proximalen Bereichs eines Schafts. Die Ausnehmung zum Aufnehmen eines proximalen Bereichs eines Schafts und die Ausnehmung zum Aufnehmen eines proximalen Bereichs einer Übertragungseinrichtung gehen insbesondere ineinander über. Der Querschnitt der Ausnehmung zum Aufnehmen des proximalen Bereichs des Schafts ist insbesondere an den Querschnitt des proximalen Bereichs des Schafts angepasst. Der Querschnitt der Ausnehmung zum Aufnehmen eines proximalen Bereichs eines Schafts ist insbesondere größer als der Querschnitt der Ausnehmung zum Aufnehmen eines proximalen Bereichs einer Übertragungseinrichtung.

Die Kontaktierungseinrichtung ist insbesondere in der Ausnehmung zum Aufnehmen eines proximalen Bereichs einer Übertragungseinrichtung oder in einer beispielsweise ringförmigen Erweiterung der Ausnehmung angeordnet. Eine Kontaktfläche der Kontaktierungseinrichtung ist ein Oberflächenbereich der Kontaktierungseinrichtung, der bei der vorgesehenen Verwendung der Handhabungseinrichtung, insbesondere bei der vorgesehenen Anordnung einer zur Kombination mit der Handhabungseinrichtung vorgesehenen Übertragungseinrichtung in der Ausnehmung der Handhabungseinrichtung unmittelbar an einem korrespondierenden Oberflächenbereich der Übertragungseinrichtung anliegt. Die Kontaktflächen der Kontaktierungseinrichtung sind voneinander beabstandet.

Die Handhabungseinrichtung ist insbesondere für eine vorbestimmte Bauform einer Übertragungseinrichtung oder für eine Übertragungseinrichtung mit vorbestimmten Eigenschaften zumindest des proximalen Bereichs der Übertragungseinrichtung vorgesehen und ausgebildet und an diese Bauform oder Eigenschaften angepasst. Die Handhabungseinrichtung ist insbesondere für eine vorbestimmte Bauform eines Schafts oder für einen Schaft mit vorbestimmten Eigenschaften zumindest des proximalen Bereichs des Schafts vorgesehen und ausgebildet und an diese Bauform oder Eigenschaften angepasst.

Mehrere Kontaktflächen der Kontaktierungseinrichtung der Handhabungseinrichtung können die Zuverlässigkeit des elektrischen Kontakts zu der Übertragungseinrichtung verbessern und den elektrischen Kontaktwiderstand reduzieren. Selbst wenn eine der Kontaktflächen korrodiert, verformt oder auf andere Weise beschädigt ist, können weitere Kontaktflächen der Kontaktierungseinrichtung eine zuverlässige elektrische Kontaktierung der Übertragungseinrichtung ermöglichen.

Bei einer Handhabungseinrichtung, wie sie hier beschrieben ist, können insbesondere alle Kontaktflächen dazu ausgebildet sein, in radialer Richtung an einer Oberfläche einer in der Ausnehmung angeordneten Übertragungseinrichtung anzuliegen.

Eine Richtung ist radial, wenn sie orthogonal zu der Längsachse einer in der vorgesehenen Weise in der Ausnehmung der Handhabungseinrichtung angeordneten Übertragungseinrichtung ist. Eine Kontaktfläche liegt in radialer Richtung an der Oberfläche der Übertragungseinrichtung an, wenn die Normalkraft zwischen der Kontaktfläche und der Oberfläche der Übertragungseinrichtung und somit die Flächennormalen der Kontaktfläche und der Oberfläche der Übertragungseinrichtung in radialer Richtung orientiert sind.

Bei einer Handhabungseinrichtung, wie sie hier beschrieben ist, können insbesondere die Flächennormalen aller Kontaktflächen radial orientiert sein.

In radialer Richtung anliegende Kontaktflächen oder Kontaktflächen, deren Flächennormalen in radialer Richtung angeordnet sind, üben auf eine in der vorgesehenen Weise in der Ausnehmung der Handhabungseinrichtung angeordnete Übertragungseinrichtung keine axiale oder zur Längsachse der Übertragungseinrichtung parallele Kraft und kein Drehmoment um die Längsachse der Übertragungseinrichtung aus. Die Kontaktflächen beeinflussen oder behindern deshalb die Übertragung einer Kraft oder eines Drehmoments mittels der Übertragungseinrichtung nicht.

Bei einer Handhabungseinrichtung, wie sie hier beschrieben ist, können insbesondere alle Kontaktflächen elektrisch parallel geschaltet sein.

Mehrere elektrisch parallel geschaltete Kontaktflächen können die Zuverlässigkeit des elektrischen Kontakts verbessern und den Kontaktwiderstand reduzieren.

Bei einer Handhabungseinrichtung, wie sie hier beschrieben ist, können insbesondere alle Kontaktflächen der Kontaktierungseinrichtung eine Ebene orthogonal zu der Längsachse einer in der vorgesehenen Weise in der Ausnehmung angeordneten Übertragungseinrichtung schneiden.

Eine Anordnung der Kontaktflächen derart, dass alle Kontaktflächen eine Ebene orthogonal zu der Längsachse der Übertragungseinrichtung schneiden, kann eine Erzeugung eines Drehmoments um eine Achse orthogonal zu der Längsachse der Übertragungseinrichtung durch die Andruckkräfte der Kontaktflächen vermeiden. Ferner kann eine derartige Anordnung der Kontaktflächen den Bauraum der Kontaktierungseinrichtung in Richtung parallel zu der Längsachse der Übertragungseinrichtung verkürzen.

Bei einer Handhabungseinrichtung, wie sie hier beschrieben ist, können Winkelabstände zwischen benachbarten Kontaktflächen der Kontaktierungseinrichtung insbesondere gleich sein.

Insbesondere sind alle Winkelabstände zwischen jeweils zwei benachbarten Kontaktflächen der Kontaktierungseinrichtung gleich. Die Winkel sind dabei immer auf die Längsachse einer in der vorgesehenen Weise in der Ausnehmung der Handhabungseinrichtung angeordneten Übertragungseinrichtung bezogen.

Bei einer Handhabungseinrichtung, wie sie hier beschrieben ist, können insbesondere zwei Kontaktflächen in gegenseitigen Winkelabständen von 180 Grad oder drei Kontaktflächen in gegenseitigen Winkelabständen von 120 Grad oder vier Kontaktflächen in gegenseitigen Winkelabständen von 90 Grad oder fünf Kontaktflächen in gegenseitigen Winkelabständen von 72 Grad angeordnet sein.

Alle Winkel sind auf die Längsachse einer in der vorgesehenen Weise in der Ausnehmung angeordneten und durch die Kontaktierungseinrichtung der Handhabungseinrichtung kontaktierten Übertragungseinrichtung bezogen. Mit dem Winkelabstand zweier benachbarter Kontaktflächen ist der Winkelabstand zwischen den Flächenschwerpunkten der Kontaktflächen gemeint.

Eine über den Umfang der Übertragungseinrichtung gleichförmig verteilte Anordnung der Kontaktflächen kann bewirken, dass die von den Kontaktflächen auf die Übertragungseinrichtung ausgeübten Kräfte einander aufheben.

Bei einer Handhabungseinrichtung, wie sie hier beschrieben ist, kann die Kontaktierungseinrichtung insbesondere monolithisch ausgebildet sein.

Die Kontaktierungseinrichtung ist beispielsweise durch Gießen, Fräsen, Ätzen oder auf andere Weise aus einem einzigen Werkstück (insbesondere aus Metall) gebildet.

Bei einer Handhabungseinrichtung, wie sie hier beschrieben ist, können insbesondere alle Kontaktflächen an ein und demselben monolithisch ausgebildeten Element ausgebildet sein.

Bei einer Handhabungseinrichtung, wie sie hier beschrieben ist, kann insbesondere mindestens eine der Kontaktflächen in einer im Wesentlichen radialen Richtung bewegbar sein. Insbesondere ist jede Kontaktfläche der Kontaktierungseinrichtung in einer im Wesentlichen radialen Richtung bewegbar. Eine Kontaktfläche ist in einer im Wesentlichen radialen Richtung bewegbar, wenn die Kontaktfläche oder ihr Flächenschwerpunkt entlang eines Pfads bewegbar ist, der mit einer Geraden, die durch den Flächenschwerpunkt der Kontaktfläche geht und orthogonal zu der Längsachse einer in der vorgesehenen Weise in der Ausnehmung angeordneten Übertragungseinrichtung orthogonal ist, einen kleinen Winkel einschließt. Ein Winkel ist klein, wenn er nicht mehr als 30 Grad oder nicht mehr als 20 Grad oder nicht mehr als 10 Grad oder nicht mehr als 5 Grad beträgt. Die Kontaktfläche oder die Kontaktflächen sind insbesondere gegen eine Rückstellkraft einer elastischen Einrichtung in einer im Wesentlichen radialen Richtung bewegbar.

Bei einer Handhabungseinrichtung, wie sie hier beschrieben ist, weist die Kontaktierungseinrichtung insbesondere mehrere Federzungen auf, wobei jede Kontaktfläche an einer Federzunge angeordnet ist.

Insbesondere ist an jeder Federzunge eine einzige Kontaktfläche ausgebildet. Jede Kontaktfläche ist insbesondere an einem freien Ende einer Federzunge angeordnet, deren anderes Ende mit der übrigen Kontaktierungseinrichtung starr verbunden ist.

Bei einer Handhabungseinrichtung, wie sie hier beschrieben ist, erstrecken die Federzungen der Kontaktierungseinrichtung sich insbesondere jeweils parallel oder im Wesentlichen parallel zu einer Ebene orthogonal zu der Längsachse einer in der vorgesehenen Weise in der Ausnehmung angeordneten Übertragungseinrichtung.

Federzungen, die sich jeweils parallel zu einer Ebene orthogonal zu der Längsachse der Übertragungseinrichtung erstrecken, können eine besonders kompakte Gestalt der Kontaktierungseinrichtung, insbesondere eine in Richtung parallel zu der Längsachse der Übertragungseinrichtung besonders kurze Ausgestaltung der Kontaktierungseinrichtung ermöglichen.

Bei einer Handhabungscinrichtung, wie sie hier beschrieben ist, können die Federzungen der Kontaktierungseinrichtung jeweils insbesondere zumindest abschnittsweise kreisbogenförmig oder im wesentlichen kreisbogenförmig ausgebildet sein.

Eine kreisbogenförmige Ausgestaltung der Federzungen kann eine kompakte Bauform der Kontaktierungseinrichtung ermöglichen.

Bei einer Handhabungseinrichtung, wie sie hier beschrieben ist, weist insbesondere jede Kontaktfläche eine Länge auf, die wesentlich größer ist als ihre Breite.

Die Länge einer Kontaktfläche wird in Richtung parallel zu der Längsachse einer in der vorgesehenen Weise in der Ausnehmung angeordneten Übertragungseinrichtung gemessen. Die Breite einer Kontaktfläche wird in einer dazu orthogonalen Richtung, insbesondere in Richtung des Umfangs einer in der vorgesehenen Weise in der Ausnehmung angeordneten Übertragungseinrichtung gemessen. Die Länge der Kontaktfläche ist insbesondere mindestens um einen Faktor fünf oder mindestens um einen Faktor zehn oder mindestens um einen Faktor zwanzig größer als ihre Breite. Mit den Abmessungen der Kontaktflächen sind insbesondere deren Abmessungen unmittelbar nach deren Fertigstellung und vor Auslieferung und Gebrauch gemeint.

Bei einer Handhabungseinrichtung, wie sie hier beschrieben ist, kann die Kontaktierungseinrichtung insbesondere ferner zum Zentrieren eines proximalen Bereichs einer in der vorgesehenen Weise in die Handhabungseinrichtung eingeführten Übertragungseinrichtung vorgesehen und ausgebildet sein.

Die Kontaktierungseinrichtung zentriert den proximalen Bereich der Übertragungseinrichtung insbesondere insofern, als sie ihn bezogen auf radiale Bewegungen führt. Die Kontaktierungseinrichtung setzt insbesondere jeder Abweichung der Übertragungseinrichtung von einer Soll-Position eine elastische Kraft entgegen. Diese Kraft wird beispielsweise durch die beschriebenen Federzungen erzeugt.

Ein medizinisches Instrument umfasst eine Handhabungseinrichtung, wie sie hier beschrieben ist, einen Schaft, dessen proximales Ende mit der Handhabungseinrichtung mechanisch verbunden oder verbindbar ist, und eine Übertragungseinrichtung zum Übertragen von elektrischer Leistung und zumindest entweder einer Kraft oder eines Drehmoments von der Handhabungseinrichtung zu einem distalen Ende eines mit der Handhabungseinrichtung zu bildenden mikroinvasiven medizinischen Instruments.

Ein proximaler Bereich des Schafts kann in einer korrespondierenden Ausnehmung der Handhabungseinrichtung angeordnet sein oder angeordnet werden. Ein proximaler Bereich der Übertragungseinrichtung kann in der korrespondierenden für die Übertragungseinrichtung vorgesehene Ausnehmung in der Handhabungseinrichtung angeordnet sein.

### Kurzbeschreibung der Figuren

Nachfolgend werden Ausführungsformen anhand der beigefügten Figuren näher erläutert. Es zeigen:
- Figur 1: eine schematische Darstellung eines Schnitts durch Komponenten eines medizinischen Instruments;
- Figur 2: eine weitere schematische Darstellung eines Schnitts durch die Komponenten des medizinischen Instruments aus Figur 1;
- Figur 3: eine schematische Darstellung eines weiteren Schnitts durch das medizinische Instrument aus den Figuren 1 und 2;
- Figur 4: eine schematische Darstellung eines Schnitts durch eine Handhabungseinrichtung;
- Figur 5: eine schematische Darstellung eines Schnitts durch eine weitere Handhabungseinrichtung.

### Beschreibung der Ausführungsformen

Figur 1 zeigt eine schematische Darstellung eines Schnitts durch Komponenten eines mikroinvasiven medizinischen Instruments 10. Insbesondere sind in Figur 1 Komponenten, die einen proximalen Bereich 12 des Instruments 10 bilden, gezeigt. Das distale Ende des Instruments 10 mit einem Werkzeug oder einer anderen Wirkeinrichtung zum Greifen, Quetschen, Halten, Kauterisieren, Schneiden, Stanzen und/oder für eine andere Wirkung befindet sich außerhalb, nämlich links des dargestellten Bereichs. Die in Figur 1 dargestellte

Schnittebene ist parallel zu einer in Figur 1 horizontal angeordneten Längsrichtung des Instruments 10.

Das Instrument 10 umfasst in seinem proximalen Bereich 12 eine Handhabungseinrichtung 20 mit einem feststehenden Bauteil 24, an dem ein elektrischer Steckkontakt 25 zur Zuführung elektrischer Leistung für elektrochirurgische Maßnehmen angeordnet ist. Ferner umfasst die Handhabungseinrichtung 20 ein manuell bewegbares Bauteil 26, das relativ zu dem feststehenden Bauteil 24 um eine Schwenkachse orthogonal zu der Zeichenebene der Figur 1 schwenkbar ist. Das feststehende Bauteil 24 und das bewegbare Bauteil 26 weisen jeweils ein Auge auf, durch das ein oder mehrere Finger einer Hand geführt werden können.

Das Instrument 10 umfasst ferner einen Schaft 60 mit einem proximalen Ende 62 und eine Übertragungseinrichtung 70 zum Übertragen elektrischer Leistung und/oder eines elektrischen Signals sowie einer Kraft und/oder eines Drehmoments zu dem in Figur 1 nicht dargestellten distalen Ende des Instruments 10. Die Übertragungseinrichtung 70 weist einen proximalen Bereich 72 auf. Das Instrument 10 ist in Figur 1 in einer Konfiguration dargestellt, in der das proximale Ende 62 des Schafts 60 und der proximale Bereich 72 der Übertragungseinrichtung 70 noch nicht mit der Handhabungseinrichtung 20 mechanisch verbunden sind und das Instrument 10 deshalb nicht verwendbar ist. Eine Längsachse 78 der Übertragungseinrichtung 70 liegt in der in Figur 1 dargestellten Schnittebene.

Die Handhabungseinrichtung 20 weist eine erste, nach distal offene Ausnehmung 46 zum Aufnehmen des proximalen Endes 62 des Schafts 60 auf. Der Querschnitt (in einer Ebene orthogonal zu der Längsachse 78 der Übertragungseinrichtung 70 und damit orthogonal zu der in Figur 1 dargestellten Schnittebene) der ersten Ausnehmung 46 und der Querschnitt des proximalen Bereichs 62 des Schafts 60 korrespondieren derart, dass der proximale Bereich 62 des Schafts 60 reibungsarm in die erste Ausnehmung 46 eingeführt werden und dann spielarm in der ersten Ausnehmung 46 gehalten sein kann. Insbesondere sind die Querschnitte der ersten Ausnehmung 46 in der Handhabungseinrichtung 20 und des proximalen Endes 62 des Schafts 60 jeweils kreisförmig oder im Wesentlichen kreisförmig, so dass der Schaft 60 relativ zu der Handhabungseinrichtung 20 um die Längsachse 78 der Übertragungseinrichtung 70 rotiert werden kann. Eine Arretierungseinrichtung zum Arretieren des proximalen Endes 62 des Schafts 60 in der ersten Ausnehmung 46 ist in Figur 1 nicht dargestellt.

Die Handhabungseinrichtung 20 weist ferner eine zweite Ausnehmung 47 auf, die proximal der ersten Ausnehmung 46 angeordnet ist und sich unmittelbar an die erste Ausnehmung 46 anschließt. Der Querschnitt (in einer Ebene orthogonal zu der Längsachse 78/ der Übertragungseinrichtung 70) der zweiten Ausnehmung 47 und der Querschnitt des proximalen Bereichs 78 der Übertragungseinrichtung 70 korrespondieren derart, dass der proximale Bereich 78 der Übertragungseinrichtung 70 ausgehend von der in Figur 1 dargestellten Konfiguration in die zweite Ausnehmung 47 eingeführt werden kann.

Die Handhabungseinrichtung 20 weist ferner eine Kontaktierungseinrichtung 50 mit Kontaktbereichen 56 auf. Die Kontaktierungseinrichtung 50 ist in einer im Wesentlichen ringförmigen Erweiterung der zweiten Ausnehmung 47 angeordnet. In der in Figur 1 dargestellten Schnittebene ist jeder Kontaktbereich 56 durch einen Spalt 54 von der übrigen Kontaktierungseinrichtung 50 getrennt. Jeder Kontaktbereich 56 bildet eine Kontaktfläche 57 zur elektrischen Kontaktierung eines korrespondierenden Oberflächenbereichs 75 des proximalen Bereichs 72 der Übertragungseinrichtung 70. Die Kontaktierungseinrichtung 50 ist elektrisch leitfähig mit dem Steckkontakt 25 an der Handhabungseinrichtung 20 verbunden.

Figur 2 zeigt eine schematische Darstellung eines Schnitts durch den proximalen Bereich 12 des anhand der Figur 1 dargestellten medizinischen Instruments 10. Die Schnittebene der Figur 2 entspricht der Schnittebene der Figur 1.

Die in Figur 2 dargestellten Konfiguration unterscheidet sich von der anhand der Figur 1 dargestellten Konfiguration des Instruments 10 dadurch, dass der proximale Bereich 62 des Schafts 60 in die erste Ausnehmung 46 der Handhabungseinrichtung 20 und der proximale Bereich 72 der Übertragungseinrichtung 70 in die zweite Ausnehmung 47 der Handhabungseinrichtung 20 eingeführt sind. Der proximale Bereich 62 des Schafts 60 ist durch die erwähnte, in den Figuren 1 und 2 nicht dargestellte Verriegelungseinrichtung in der ersten Ausnehmung 46 der Handhabungseinrichtung 20 arretiert.

Bei der in Figur 2 gezeigten Konfiguration ist das proximale Ende der Übertragungseinrichtung 70 mit dem bewegbaren Bauteil 26 der Handhabungseinrichtung 20 derart mechanisch gekoppelt, dass eine Schwenkbewegung des bewegbaren Bauteils 26 relativ zu dem feststehenden Bauteil 24 der Handhabungseinrichtung 20 mit einer Translationsbewegung der Übertragungseinrichtung 70 relativ zu dem Schaft 60 parallel zu der Längsachse 78 der Übertragungseinrichtung 70 einhergeht.

Bei der in Figur 2 gezeigten Konfiguration liegen die Kontaktflächen 57 der Kontaktbereiche 56 der Kontaktierungseinrichtung 50 an mindestens zwei, bei dem dargestellten Beispiel einander gegenüberliegenden Stellen an dem Oberflächenbereich 75 des proximalen Bereichs 72 der Übertragungseinrichtung 70 an und stellen so einen elektrischen Kontakt her.

Figur 3 zeigt eine schematische Darstellung eines weiteren Schnitts durch das anhand der Figuren 1 und 2 dargestellte Instrument 10 in einer beispielhaften Ausgestaltung der Kontaktierungseinrichtung 50. Die Schnittebene III-III der Figur 3 ist orthogonal zu der Längsachse 78 der Übertragungseinrichtung 70 und orthogonal zu den Schnittebenen der Figuren 1 und 2. Die Schnittebene III-III schneidet die Kontaktierungseinrichtung 50. Die Position der Schnittebene III-III der Figur 3 ist in Figur 2 angedeutet.

Die Kontaktierungseinrichtung 50 weist in ihrer in Figur 3 dargestellten Ausgestaltung vier Kontaktbereiche 56 auf. Jeder Kontaktbereich 56 weist in der Schnittebene III-III der Figur 3 einen im Wesentlichen kreisförmigen Querschnitt auf und ist an einem freien Ende einer im Wesentlichen kreisbogenförmigen Federzunge 52 angeordnet. Jede Federzunge 52 ist durch einen Spalt 54 von einem ringförmigen Bereich 51 der Kontaktierungseinrichtung 50 getrennt. Die Kontaktbereiche 56 und die Federzungen 52 sind innerhalb des ringförmigen Bereichs 51 angeordnet. Das von dem Kontaktbereich 56 abgewandte Ende jeder Federzunge 52 ist mit dem ringförmigen Bereich 51 der Kontaktierungseinrichtung 50 mechanisch starr verbunden.

Die Kontaktierungseinrichtung 50 ist monolithisch ausgebildet, d. h. de ringförmige Bereich 51, die Federzungen 52 und die Kontaktbereiche 56 sind aus einem einzigen Werkstück aus einem Metall oder einem anderen elektrisch leitfähigen Material durch Gießen, Fräsen, Schneiden, Ätzen oder auf andere Weise gebildet.

Durch die kreisförmigen Querschnitte der Kontaktbereiche 56 und den kreisförmigen Querschnitt des proximalen Bereichs 72 der Übertragungseinrichtung 70 sind die an dem kreiszylindrischen Oberflächenbereich 75 der Übertragungseinrichtung 70 unmittelbar anliegenden Kontaktflächen 57 (vgl. Figuren 1, 2) linienförmig und in dem in Figur 3 dargestellten Querschnitt punktförmig. Eine Abnutzung der Kontaktbereiche 56 kann eine Verbreiterung der Kontaktflächen bewirken.

Figur 4 zeigt eine schematische Darstellung eines Schnitts durch eine Handhabungseinrichtung 20 für ein medizinisches Instrument, die in einigen Merkmalen, Eigenschaften und Funktionen der Handhabungseinrichtung 20 des anhand der Figuren 1 bis 3 dargestellten Instruments ähnelt. Die Schnittebene der Figur 4 entspricht der Schnittebene der Figur 3. Nachfolgend sind insbesondere Merkmale, Eigenschaften und Funktionen beschrieben, in denen die in Figur 4 gezeigte Handhabungseinrichtung 20 sich von den anhand der Figuren 1 bis 3 dargestellten Handhabungseinrichtungen unterscheidet.

Die Kontaktierungseinrichtung 50 der in Figur 4 gezeigten Handhabungseinrichtung 20 weist drei Kontaktbereiche mit je einer Kontaktfläche 57 auf. Die Kontaktbereiche 56 sind gleichmäßig über den in Figur 4 durch eine gestrichelte Linie angedeuteten Umfang einer in die Handhabungseinrichtung 20 in der vorgesehenen Weise eingeführten Übertragungseinrichtung verteilt. Der Winkel zwischen jeweils zwei benachbarten Kontaktbereichen 56 beträgt 120 Grad.

Die Kontaktierungseinrichtung 50 der in Figur 4 gezeigten Handhabungseinrichtung 20 unterscheidet sich von der Kontaktierungseinrichtung der anhand der Figuren 1 bis 3 dargestellten Handhabungseinrichtung ferner dadurch, dass die Querschnitte der Kontaktbereiche 56 in der Schnittebene der Figur 4 näherungsweise dreieckig sind. Jeder einzelne Kontaktbereiche 56 weist somit - zumindest in der Umgebung seiner Kontaktflächen 57 - eine prismatische Gestalt mit dreieckigem Querschnitt auf.

Figur 5 zeigt eine schematische Darstellung eines Schnitts durch eine weitere Handhabungseinrichtung 20, die in einigen Merkmalen, Eigenschaften und Funktionen den anhand der Figuren 1 bis 4 dargestellten Handhabungseinrichtungen ähnelt. Die Schnittebene der Figur 5 entspricht den Schnittebenen der Figuren 3 und 4. Nachfolgend sind insbesondere Merkmale, Eigenschaften und Funktionen der in Figur 5 gezeigten Handhabungseinrichtung dargestellt, in denen diese sich von den anhand der Figuren 1 bis 4 dargestellten Handhabungseinrichtungen unterscheidet.

Die in Figur 5 gezeigte Handhabungseinrichtung unterscheidet sich von den anhand der Figuren 1 bis 4 dargestellten Handhabungseinrichtungen insbesondere dadurch, dass die Kontaktierungseinrichtung 50 zwei Kontaktbereiche 56 aufweist. Die Kontaktbereiche 56 sind einander gegenüber angeordnet, weisen also einen Winkelabstand von 180 Grad auf.

Die in Figur 5 gezeigte Handhabungseinrichtung 20 unterscheidet sich von den anhand der Figuren 1 bis 4 dargestellten Handhabungseinrichtungen ferner dadurch, dass die Kontaktflächen 57 nicht linienförmig oder schmal ausgebildet sind. Die Kontaktfläche 57 jedes Kontaktbereichs 56 weist vielmehr eine in Richtung Breite - gemessen parallel zu dem in Figur 5 durch eine gestrichelte Linie angedeuteten Umfang einer in die Handhabungseinrichtung 20 eingesetzten Übertragungseinrichtung 70 - auf, die insbesondere nicht oder nicht deutlich kleiner ist als die in Richtung parallel zu der Längsachse der Übertragungseinrichtung 70 gemessene Länge.

Merkmale der verschiedenen Ausgestaltungen der Kontaktierungseinrichtung 50 sind in anderer Weise miteinander kombinierbar. Insbesondere kann eine Kontaktierungseinrichtung 50 zwei, drei, fünf oder mehr Kontaktbereiche 56 mit jeweils kreisförmigem Querschnitt (wie anhand der Figur 3 dargestellt) oder zwei, vier oder mehr Kontaktbereiche 56 mit dreieckigem Querschnitt (wie anhand der Figur 4 dargestellt) oder drei oder mehr Kontaktbereiche 56 mit einer breiten, nicht nur im Wesentlichen linienförmigen Kontaktfläche (wie anhand der Figur 5 dargestellt) aufweisen. Ferner kann eine Kontaktierungseinrichtung 50 mehrere Kontaktbereiche 56 mit unterschiedlichen Querschnitten aufweisen.

Die Querschnitte der Kontaktbereiche 56 können über ihre gesamte (in Richtung parallel zu der Längsachse 78 einer in der vorgesehenen Weise mit der Handhabungseinrichtung gekoppelten Übertragungseinrichtung gemessene) Länge konstant sein. Alternativ können die Querschnitte der Kontaktbereiche 56 über ihre Länge variieren. Insbesondere können die Kontaktbereiche 56 - wie in den Figuren 1 und 2 angedeutet - nach distal Rampen oder schräge Flächen oder Konturen aufweisen, die ein Einführen einer Übertragungseinrichtung in die Kontaktierungseinrichtung 50 vereinfachen können.

### Bezugszeichen

- 10: mikroinvasives medizinisches Instrument
- 12: proximaler Bereich des mikroinvasiven medizinischen Instruments 10
- 20: Handhabungseinrichtung des mikroinvasiven medizinischen Instruments 10
- 24: feststehendes Bauteil der Handhabungseinrichtung 20, zur mechanischen Verbindung mit dem proximalen Bereich 62 des Schafts 60
- 25: Steckkontakt an der Handhabungseinrichtung
- 26: manuell bewegbares Bauteil der Handhabungseinrichtung 20, zur mechanischen Kopplung mit dem proximalen Bereich 72 der Übertragungseinrichtung 70
- 46: erste Ausnehmung in dem feststehenden Bauteil 24, zur Aufnahme des proximalen Bereichs 62 des Schafts 60
- 47: zweite Ausnehmung in dem feststehenden Bauteil 24, zur Aufnahme des proximalen Bereichs 72 der Übertragungseinrichtung 70
- 50: Kontaktierungseinrichtung in der zweiten Ausnehmung 40, zur elektrischen Kontaktierung des Oberflächenbereichs 75 des proximalen Bereichs 72 der Übertragungseinrichtung 70
- 51: ringförmiger Bereich der Kontaktierungseinrichtung 50
- 52: Federzunge der Kontaktierungseinrichtung 50
- 54: Spalt zwischen der Federzunge 52 und der übrigen Kontaktierungseinrichtung 50
- 56: Kontaktbereich der Kontaktierungseinrichtung 50
- 57: Kontaktfläche an dem Kontaktbereich 56
- 60: Schaft des mikroinvasiven medizinischen Instruments
- 62: proximaler Bereich des Schafts 60
- 70: Übertragungseinrichtung des mikroinvasiven medizinischen Instruments
- 72: proximaler Bereich der Übertragungseinrichtung 70
- 75: zur Kontaktierung durch die Kontaktflächen 57 vorgesehener Oberflächenbereich der Übertragungseinrichtung 70
- 78: Längsachse der Übertragungseinrichtung 70

## Patentansprüche

1. **Handhabungseinrichtung** (20) für ein mikroinvasives medizinisches Instrument (10), mit:
einer **Ausnehmung** (47) zum Aufnehmen eines proximalen Bereichs (72) einer elektrisch leitfähigen Übertragungseinrichtung (70) zum Übertragen von elektrischer Leistung und zumindest entweder einer Kraft oder eines Drehmoments zu einem distalen Ende eines mikroinvasiven medizinischen Instruments (10);
einer **Kontaktierungseinrichtung** (50) zum Herstellen eines elektrischen Kontakts zu einer in der Ausnehmung (47) angeordneten Übertragungseinrichtung (70),
wobei die Kontaktierungseinrichtung (50) **mehrere Kontaktflächen** (57) zum gleichzeitigen Anliegen an einer Oberfläche (75) einer in der vorgesehenen Weise in der Ausnehmung (47) angeordneten Übertragungseinrichtung (70) aufweist,
**dadurch gekennzeichnet, dass**
bei der die Kontaktierungseinrichtung (50) mehrere **Federzungen** (52) aufweist,
jede Kontaktfläche (57) an einer Federzunge (52) angeordnet ist, und
sich die **Federzungen** (52) der Kontaktierungseinrichtung (50) jeweils **parallel** oder im Wesentlichen parallel **zu einer Ebene orthogonal** zu der Längsachse (78) der Übertragungseinrichtung (70) erstrecken und
jede **Kontaktfläche** (57) eine **Länge** aufweist, die wesentlich **größer** ist **als** ihre **Breite.**

2. Handhabungseinrichtung (20) nach dem vorangehenden Anspruch, bei der die Kontaktflächen (57) der Kontaktierungseinrichtung (50) **eine Ebene orthogonal** zu der Längsachse (78) einer in der vorgesehenen Weise in der Ausnehmung (47) angeordneten Übertragungseinrichtung (70) schneiden.

3. Handhabungseinrichtung (20) nach einem der vorangehenden Ansprüche, bei der **Winkelabstände** zwischen benachbarten Kontaktflächen (57) der Kontaktierungseinrichtung (50) **gleich** sind.

4. Handhabungseinrichtung (20) nach einem der vorangehenden Ansprüche, bei der zwei Kontaktflächen (57) in gegenseitigen Winkelabständen von **180 Grad** oder **drei** Kontaktflächen (57) in gegenseitigen Winkelabständen von **120 Grad** oder **vier** Kontaktflächen (57) in gegenseitigen Winkelabständen von **90 Grad** oder **fünf** Kontaktflächen (57) in gegenseitigen Winkelabständen von **72 Grad** angeordnet sind.

5. Handhabungseinrichtung (20) nach einem der vorangehenden Ansprüche, bei der die Kontaktierungseinrichtung (50) **monolithisch** ausgebildet ist.

6. Handhabungseinrichtung (20) nach Anspruch 5 , bei der die **Federzungen** (52) der Kontaktierungseinrichtung (50) jeweils zumindest abschnittsweise **kreisbogenförmig** oder im Wesentlichen kreisbogenförmig ausgebildet sind.

7. Handhabungseinrichtung (20) nach einem der vorangehenden Ansprüche, bei der die Kontaktierungseinrichtung (50) ferner zum **Zentrieren** eines proximalen Bereichs (72) einer in der vorgesehenen Weise in die Handhabungseinrichtung (20) eingeführten Übertragungseinrichtung (70) vorgesehen und ausgebildet ist.

8. **Medizinisches Instrument** (10) mit:
einem **Handhabungseinrichtung** (20) nach einem der vorangehenden Ansprüche;
einem Schaft (60), dessen proximales Ende (62) mit der Handhabungseinrichtung (20) mechanisch verbunden oder verbindbar ist;
einer **Übertragungseinrichtung** (70) zum Übertragen von elektrischer Leistung und zumindest entweder einer Kraft oder eines Drehmoments von der Handhabungseinrichtung (20) zu einem distalen Ende eines mit der Handhabungseinrichtung (20) zu bildenden mikroinvasiven medizinischen Instruments (10).

## Claims

1. Manipulation device (20) for a microinvasive medical instrument (10), having:
a recess (47) for receiving a proximal region (72) of an electrically conductive transmission device (70) for transmitting electrical power and at least either a force or a torque to a distal end of a microinvasive medical instrument (10);
a contacting device (50) for producing an electrical contact to a transmission device (70) arranged in the recess (47),
wherein the contacting device (50) has a plurality of contact faces (57) for simultaneous abutment against a surface (75) of a transmission device (70) arranged in the intended manner in the recess (47),
**characterized in that**
in which the contacting device (50) has a plurality of resilient tongues (52),
each contact face (57) is arranged at a resilient tongue (52), and
the resilient tongues (52) of the contacting device (50) each extend parallel or substantially parallel to a plane orthogonal to the longitudinal axis (78) of the transmission device (70), and
each contact face (57) has a length which is significantly larger than its width.

2. Manipulation device (20) according to the preceding claim, in which the contact faces (57) of the contacting device (50) intersect a plane orthogonal to the longitudinal axis (78) of a transmission device (70) arranged in the intended manner in the recess (47).

3. Manipulation device (20) according to either of the preceding claims, in which angular spacings between adjacent contact faces (57) of the contacting device (50) are equal.

4. Manipulation device (20) according to one of the preceding claims, in which two contact faces (57) are arranged with mutual angular spacings of 180 degrees, or three contact faces (57) are arranged with mutual angular spacings of 120 degrees, or four contact faces (57) are arranged with mutual angular spacings of 90 degrees, or five contact faces (57) are arranged with mutual angular spacings of 72 degrees.

5. Manipulation device (20) according to one of the preceding claims, in which the contacting device (50) is of monolithic form.

6. Manipulation device (20) according to Claim 5, in which the resilient tongues (52) of the contacting device (50) are each formed at least sectionally in the shape of an arc of a circle or substantially in the shape of an arc of a circle.

7. Manipulation device (20) according to one of the preceding claims, in which the contacting device (50) is furthermore provided and configured for centring a proximal region (72) of a transmission device (70) inserted in the intended manner into the manipulation device (20).

8. Medical instrument (10) having:
a manipulation device (20) according to one of the preceding claims;
a shaft (60), whose proximal end (62) is mechanically connected or connectable to the manipulation device (20);
a transmission device (70) for transmitting electrical power and at least either a force or a torque from the manipulation device (20) to a distal end of a microinvasive medical instrument (10) to be formed with the manipulation device (20).

## Revendications

1. Dispositif de manipulation (20) destiné à un instrument médical micro-invasif (10), ledit dispositif comprenant :
un évidement (47) destiné à recevoir une zone proximale (72) d'un dispositif de transmission électriquement conducteur (70) destiné à transmettre une puissance électrique et au moins une force ou un couple à une extrémité distale d'un instrument médical micro-invasif (10) ;
un dispositif de mise en contact (50) destiné à réaliser un contact électrique avec un dispositif de transmission (70) disposé dans l'évidement (47),
le dispositif de mise en contact (50) comportant une pluralité de surfaces de contact (57) destinées à venir simultanément en appui sur une surface (75) d'un dispositif de transmission (70) disposé de la manière prévue dans l'évidement (47),
**caractérisé en ce que**
dans lequel le dispositif de mise en contact (50) comporte une pluralité de languettes élastiques (52), chaque surface de contact (57) est disposée sur une languette élastique (52), et
les languettes élastiques (52) du dispositif de mise en contact (50) s'étendent chacune parallèlement ou sensiblement parallèlement à un plan orthogonal à l'axe longitudinal (78) du dispositif de transmission (70) et chaque surface de contact (57) a une longueur qui est sensiblement supérieure à sa largeur.

2. Dispositif de manutention (20) selon la revendication précédente, dans lequel les surfaces de contact (57) du dispositif de mise en contact (50) coupent un plan orthogonal à l'axe longitudinal (78) d'un dispositif de transmission (70) disposé de la manière prévue dans l'évidement (47).

3. Dispositif de manutention (20) selon l'une des revendications précédentes, dans lequel les distances angulaires entre des surfaces de contact adjacentes (57) du dispositif de mise en contact (50) sont identiques.

4. Dispositif de manutention (20) selon l'une des revendications précédentes, dans lequel deux surfaces de contact (57) sont disposées à des distances angulaires l'une de l'autre de 180 degrés ou trois surfaces de contact (57) sont disposées à des distances angulaires les unes des autres de 120 degrés ou quatre surfaces de contact (57) sont disposées à des distances angulaires les unes des autres de 90 degrés ou cinq surfaces de contact (57) sont disposées à des distances angulaires les unes des autres de 72 degrés.

5. Dispositif de manutention (20) selon l'une des revendications précédentes, dans lequel le dispositif de mise en contact (50) est monolithique.

6. Dispositif de manutention (20) selon la revendication 5, dans lequel les languettes élastiques (52) du dispositif de mise en contact (50) sont formées chacune au moins par portions en arc de cercle ou sensiblement en arc de cercle.

7. Dispositif de manipulation (20) selon l'une des revendications précédentes, dans lequel le dispositif de mise en contact (50) est également prévu et conçu pour centrer une zone proximale (72) d'un dispositif de transmission (70) introduit dans le dispositif de manipulation (20) de la manière prévue.

8. Instrument médical (10) comprenant :
un dispositif de manipulation (20) selon l'une des revendications précédentes ;
une tige (60) dont l'extrémité proximale (62) est reliée ou peut être reliée mécaniquement au dispositif de manipulation (20) ;
un dispositif de transmission (70) destiné à transmettre de la puissance électrique et au moins une force et un couple du dispositif de manipulation (20) à une extrémité distale d'un instrument médical micro-invasif (10) à former avec le dispositif de manipulation (20).
